# EUROPEAN PATENT SPECIFICATION

(11) **EP 3 243 488 B1**
(45) Date of publication and mention of the grant of the patent: **18.09.2019**
(21) Application number: 16203575.2
(22) Date of filing: 13.12.2016
(51) Int. Cl.: A61F 5/00, A61B 17/3207, A61M 25/10

(54) **MEDICAL DEVICE**
MEDIZINISCHE VORRICHTUNG
DISPOSITIF MÉDICAL

(30) Priority: 09.05.2016 JP 2016094143
(43) Date of publication of application: 15.11.2017
(73) Proprietor: Asahi Intecc Co., Ltd., Seto-shi, Aichi 489-0071 (JP)
(72) Inventor: Nishigishi, Makoto, Seto-shi, Aichi 489-0071 (JP)
(74) Representative: Winter, Brandl, Fürniss, Hübner, Röss, Kaiser, Polte - Partnerschaft mbB

(56) References cited:
- WO-A1-2009/116159
- WO-A1-2013/023121
- US-A- 5 196 024
- US-A1- 2008 228 139

## Description

### [Field]

The present invention relates to a medical device comprising an incising member for incising a stenosis or stricture in a blood vessel or in a digestive organ in the longitudinal direction.

### [Background]

Conventionally, a stenosis or stricture formed in a blood vessel or in a digestive organ is treated by a well-known method, by which method the stenosis or stricture is dilated with a medical device such as a balloon catheter. Such a medical device generally has an expandable-and-contractible member capable of radially expanding and radially contracting (a balloon or a mesh member, for example), and the expandable-and-contractible member is made expand radially to dilate the stenosis or stricture.

When the stenosis or stricture has been highly calcified, however, it is difficult to dilate it with the expandable-and-contractible member alone. So, for use for a calcified stenosis or stricture, a medical device (an incising device) is known, which has an incising member disposed therein in the longitudinal direction and is used by inserting a balloon catheter into the interior of the incising member and then making the balloon of the balloon catheter expand so as to radially expand the incising member (see US2013/0041391 or US 2008/228139).

In the medical device (the incising device) of US2013/0041391 or US 2008/228139, the incising member (a cutting element), which is disposed therein in the longitudinal direction, is directly fixed to the outer circumference of wire that constitute the expandable-and-contractible member (an expandable scaffold). Upon radial expansion, the expandable-and-contractible member (the expandable scaffold) can incise a stenosis or stricture in the longitudinal direction.

Generally, as the expandable-and-contractible member (the expandable scaffold) expands radially, it contracts in the longitudinal direction (in other words, as the expandable-and-contractible member expands, it becomes shorter). In the medical device (the incising device) described above, the incising member (the cutting element) is directly fixed to the outer circumference of the wire that constitute the expandable-and-contractible member (the expandable scaffold) and, therefore, cannot move in the longitudinal direction as the expandable-and-contractible member (the expandable scaffold) expands or contracts. Consequently, upon attempted radial expansion of the balloon of the balloon catheter inserted in the interior of the expandable-and-contractible member (the expandable scaffold), the radial expansion of the expandable-and-contractible member (the expandable scaffold) is interfered with by the incising member (the cutting element), which is a problem. In addition, when the expandable-and-contractible member (the expandable scaffold) is forcefully made expand, the incising member (the cutting element) comes off the expandable-and-contractible member (the expandable scaffold), which is another problem.

Sometimes, upon expansion of the expandable-and-contractible member (the expandable scaffold), the stenosis or stricture can be partially caught between the expandable-and-contractible member (the expandable scaffold) and the incising member (the cutting element). When the handler manipulates the medical device in the longitudinal direction, the stenosis or stricture thus caught can cause the incising member (the cutting element) to come off the expandable-and-contractible member (the expandable scaffold), which is another problem.

### [Summary]

### [Technical Problem]

The present invention is devised based on the above circumstances, and an object of the present invention is to provide a medical device in which an incising member is not likely to come off upon radial expansion or radial contraction of an expandable-and-contractible member and the incising member is not likely to interfere with the expanding or contracting movement of the expandable-and-contractible member.

### [Solution to Problem]

This object is achieved in the following way. The invention is defined by the appended claims. Subject-matter referred to as embodiments and/or inventions not being under the scope of the appended claims merely represent possible exemplary executions and are not part of the invention.

A medical device that is disclosed in the present invention comprises an expandable-and-contractible member capable of radially expanding and radially contracting; an incising member disposed on an outer circumference of the expandable-and-contractible member, being not fixed to the expandable-and-contractible member and comprising a hollow portion open toward a proximal end direction; a core wire inserted in the hollow portion of the incising member from the proximal end direction; a distal tip fixed to both a distal end of the expandable-and-contractible member and a distal end of the incising member; and a ring fixed to both a proximal end of the expandable-and-contractible member and a proximal end of the core wire, wherein the core wire is capable of sliding in a longitudinal direction within the hollow portion as the expandable-and-contractible member expands or contracts.

An embodiment of the present invention provides: the medical device according to the invention, wherein the expandable-and-contractible member is a mesh member woven from a first wire and a second wire, and wherein the core wire extends in the longitudinal direction underneath a space that is defined by the first wire and the second wire.

### [Advantageous Effects of Invention]

A medical device according to the present invention has a configuration where the distal end of an expandable-and-contractible member and the distal end of an incising member are fixed to a distal tip, the proximal end of the expandable-and-contractible member and the proximal end of a core wire are fixed to a ring, the core wire is inserted in a hollow portion of the incising member from a proximal end direction, and the core wire can slide in the longitudinal direction within the hollow portion as the expandable-and-contractible member expands or contracts. In this configuration, as the expandable-and-contractible member radially expands, part of the core wire housed within the hollow portion of the incising member becomes shorter in the longitudinal direction, while, as the expandable-and-contractible member radially contracts, the part of the core wire housed within the hollow portion of the incising member becomes longer in the longitudinal direction. Owing to the presence of the core wire inserted in the hollow portion, the incising member does not come off the expandable-and-contractible member as the expandable-and-contractible member expands or contracts and can slide to an optimum position. As a result, the possibility of the incising member interfering with the radial expansion or the radial contraction of the expandable-and-contractible member can be reduced. Even when the handler manipulates the medical device in the longitudinal direction in a state where the stenosis or stricture is partially caught between the expandable-and-contractible member and the incising member, the core wire can be pulled out of the hollow portion of the incising member (in other words, the core wire and the incising member can become separated from each other), so that the load (external force) applied on the incising member by the stenosis or stricture thus caught can be reduced, consequently reducing the possibility of the incising member coming off.

A medical device according to the above embodiment of the present invention has a configuration where an expandable-and-contractible member is a mesh member woven from a first wire and a second wire and where a core wire extends in the longitudinal direction underneath a space that is defined by the first wire and the second wire. Because of this configuration, even if the core wire is pulled out of a hollow portion of an incising member (in other words, the core wire and the incising member become separated from each other), the likelihood of the core wire moving outwardly and coming off the mesh member can be reduced, and, as a result, the possibility of the wall of a normal blood vessel or the wall of a normal digestive organ becoming impaired by the core wire pulled out of (or separated from) the incising member can be reduced.

### [Brief Description of Drawings]

FIG. 1 is an overall view of a medical device according to a first embodiment, in which an expandable-and-contractible member is radially contracted.
FIG. 2 is an overall view of the medical device according to the first embodiment, in which the expandable-and-contractible member is radially expanded.
FIG. 3 is a sectional view taken from line A-A of FIG. 1.
FIG. 4 is an overall view of a medical device according to a first example, in which an expandable-and-contractible member is radially contracted.
FIG. 5 is an overall view of the medical device according to said first example, in which the expandable-and-contractible member is radially expanded.
FIG. 6 is an overall view of a medical device according to a second embodiment, in which an expandable-and-contractible member is radially contracted.
FIG. 7 is an overall view of the medical device according to the second embodiment, in which the expandable-and-contractible member is radially expanded.
FIG. 8 is a sectional view taken from line B-B of FIG. 6.
FIG. 9 is an overall view of a medical device according to a second example, in which an expandable-and-contractible member is radially contracted.
FIG. 10 is an overall view of the medical device according to said second example, in which the expandable-and-contractible member is radially expanded.
FIG. 11 is an overall view of a medical device according to a third embodiment, in which an expandable-and-contractible member is radially contracted.
FIG. 12 is an overall view of the medical device according to the third embodiment, in which the expandable-and-contractible member is radially expanded.
FIG. 13 is an overall view of a medical device according to a third example, in which an expandable-and-contractible member is radially contracted.
FIG. 14 is an overall view of the medical device according to the third example, in which the expandable-and-contractible member is radially expanded.
FIG. 15 is an overall view of a medical device according to a fourth embodiment, in which an expandable-and-contractible member is radially expanded.
FIG. 16 is a variation of FIG. 8.

### [Description of Embodiments]

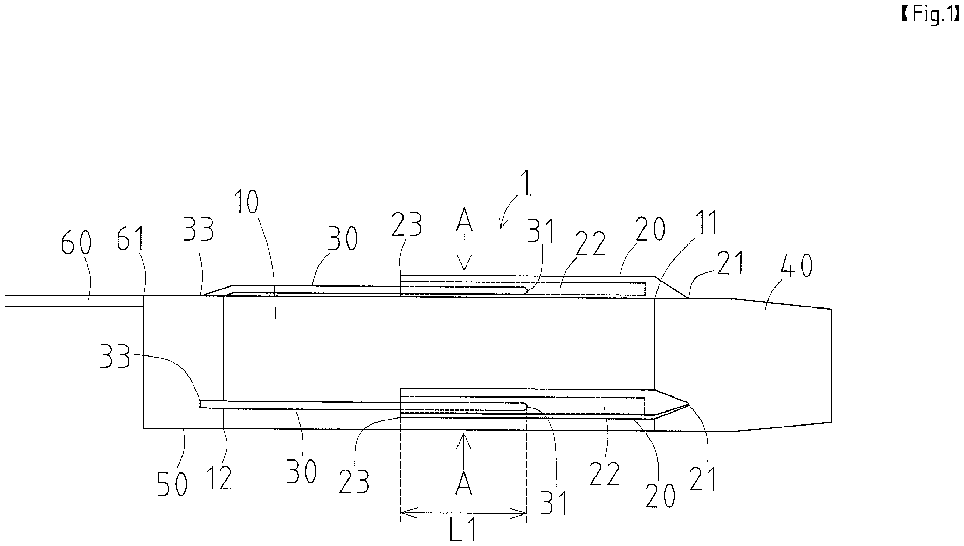

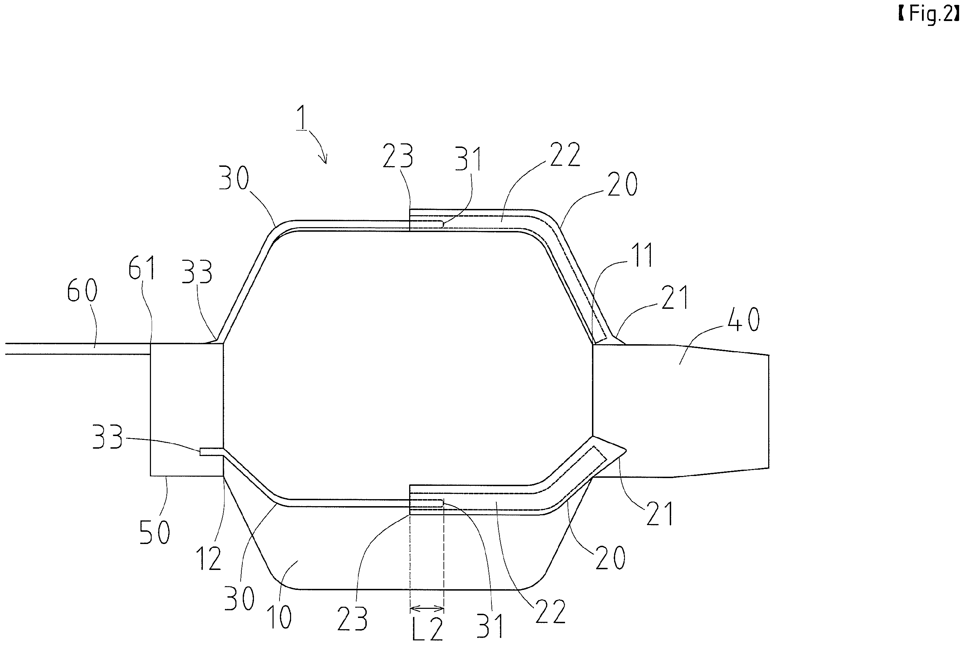

A medical device 1 according to a first embodiment is described below referring to FIG. 1 to FIG. 3. In FIG. 1 to FIG. 3, the distal end side (the front-end side) to be inserted into the body is shown to the right hand side, and the proximal end side (the rear-end side, the base-end side) to be manipulated by a handler such as a doctor is shown to the left hand side. FIG. 1 shows an expandable-and-contractible member 10 radially contracted, and FIG. 2 shows the expandable-and-contractible member 10 radially expanded.

The medical device 1 is, for example, a therapeutic device used for dilating a stenosis or stricture for curative purposes. As shown in FIG. 1 and FIG. 2, the medical device 1 essentially comprises the expandable-and-contractible member 10 capable of radially expanding and radially contracting, an incising member 20, a core wire 30, a distal tip 40, a ring 50, and a shaft 60.

The expandable-and-contractible member 10 is made of resin material and is capable of radially expanding and radially contracting. A distal end 11 of the expandable-and-contractible member 10 is fixed to the distal tip 40, and a proximal end 12 of the expandable-and-contractible member 10 is fixed to the ring 50.

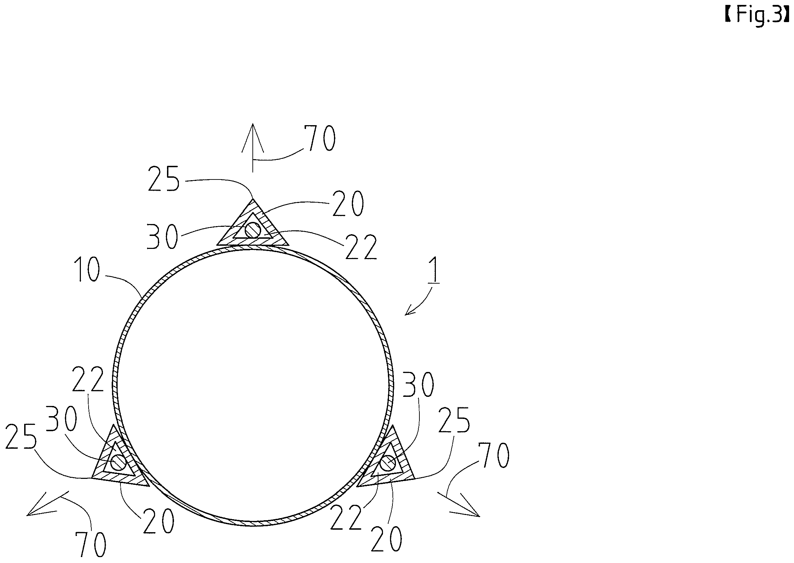

The incising member 20 is disposed in the longitudinal direction on the outer circumference of the expandable-and-contractible member 10 and comprises a hollow portion 22 that is open toward the proximal end direction. A distal end 21 of the incising member 20 is fixed to the distal tip 40, and a proximal end 23 of the incising member 20 is not fixed to the expandable-and-contractible member 10 and remains unsuspended. In other words, the incising member 20 is not fixed to the expandable-and-contractible member 10 except for its distal end 21 being fixed to the distal tip 40. In the medical device 1 according to the first embodiment, the number of the incising member 20 is three (see FIG. 3) .

The core wire 30 is disposed in the longitudinal direction on the outer circumference of the expandable-and-contractible member 10, with a distal end 31 thereof being within the hollow portion 22 of the incising member 20. As to be described below, the distal end 31 of the core wire 30 is not fixed within the hollow portion 22 of the incising member 20 and can slide in the longitudinal direction within the hollow portion 22 of the incising member 20 as the expandable-and-contractible member 10 expands or contracts. A proximal end 33 of the core wire 30 is fixed to the ring 50. In the medical device 1 according to the first embodiment, the number of the core wire 30 is three (see FIG. 3).

To the distal end 11 of the expandable-and-contractible member 10 and the distal end 21 of the incising member 20, the distal tip 40 made of resin material is fixed.

To the proximal end 12 of the expandable-and-contractible member 10 and the proximal end 33 of the core wire 30, the ring 50 made of metal material is fixed.

To the ring 50, a distal end 61 of the shaft 60 is fixed. The shaft 60 is a thin, cylinder-like metal wire (stainless steel), extending from the ring 50 in the longitudinal direction toward the proximal end side.

The handler inserts the medical device 1 into a blood vessel or a digestive organ and manipulates the shaft 60 in the longitudinal direction to deliver the medical device 1 to the site of a stenosis or stricture. During this procedure, the expandable-and-contractible member 10 is radially contracted (see FIG. 1). Subsequently, the handler inserts a balloon catheter into the interior of the expandable-and-contractible member 10 of the medical device 1 and then makes the balloon of the balloon catheter radially expand so as to radially expand the expandable-and-contractible member 10 (see FIG. 2). By pressing the incising member 20 against the stenosis or stricture, the handler can incise the stenosis or stricture in the longitudinal direction.

In FIG. 2, the balloon catheter inserted in the interior of the expandable-and-contractible member 10 is omitted for ease of understanding. The balloon catheter used in the medical device 1 can be a known balloon catheter.

FIG. 3 is a sectional view taken from line A-A of FIG. 1. On the outer circumference of the expandable-and-contractible member 10, three incising members 20 are disposed in an arrangement where they are away from each other by 120 degrees in the circumferential direction. The incising member 20 is a triangular tube with its interior being the hollow portion 22, and comprises a protruding portion 25 protruding outwardly. Upon radial expansion of the expandable-and-contractible member 10, the incising member 20 moves to the direction shown by an arrow 70, so that the protruding portion 25 of the incising member 20 can incise a stenosis or stricture.

In the medical device 1, the core wire 30, which is inserted in the hollow portion 22 of the incising member 20 from the proximal end direction, can slide in the longitudinal direction within the hollow portion 22 as the expandable-and-contractible member 10 expands or contracts. More specifically, upon radial expansion of the expandable-and-contractible member 10 (in other words, upon a shift from the state in FIG. 1 to the state in FIG. 2), part of the core wire 30 housed within the hollow portion 22 of the incising member 20 becomes shorter in the longitudinal direction from L1 to L2, while, upon radial contraction of the expandable-and-contractible member 10 (in other words, upon a shift from the state in FIG. 2 to the state in FIG. 1), the part of the core wire 30 housed within the hollow portion 22 of the incising member 20 becomes longer in the longitudinal direction from L2 to L1.

As described above, in the medical device 1, the incising member 20 is not fixed to the expandable-and-contractible member 10, and, owing to the presence of the core wire 30 inserted in the hollow portion 22, the incising member 20 does not comes off the expandable-and-contractible member 10 as the expandable-and-contractible member 10 expands or contracts and can slide to an optimum position, consequently reducing the possibility of the incising member 20 interfering with the radial expansion or the radial contraction of the expandable-and-contractible member 10. Even when the stenosis or stricture is partially caught between the expandable-and-contractible member 10 and the incising member 20 and the handler manipulates the medical device 1 in the longitudinal direction in that state, the core wire 30 can be pulled out of the hollow portion 22 of the incising member 20 (in other words, the core wire 30 and the incising member 20 can become separated from each other), so that the load (external force) applied on the incising member 20 by the stenosis or stricture thus caught can be reduced, consequently reducing the possibility of the incising member 20 coming off.

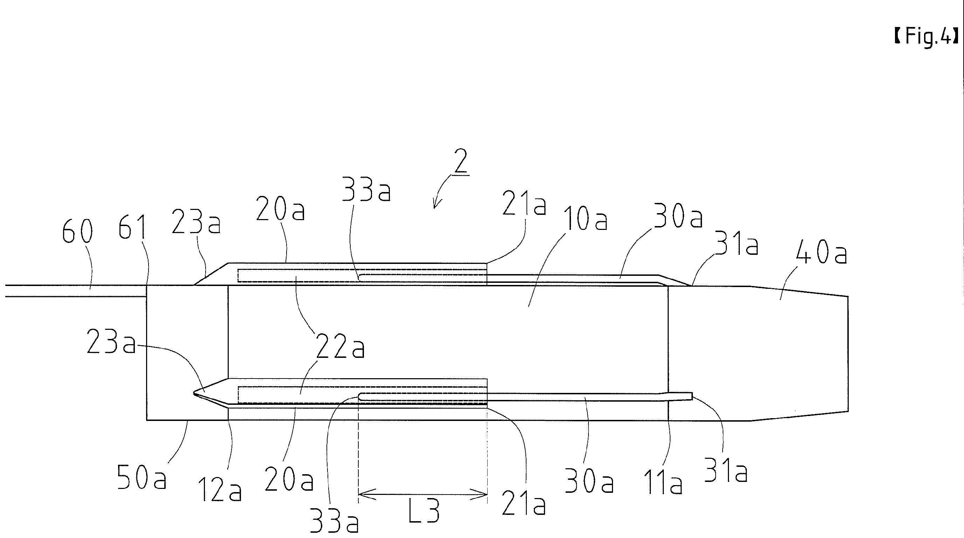

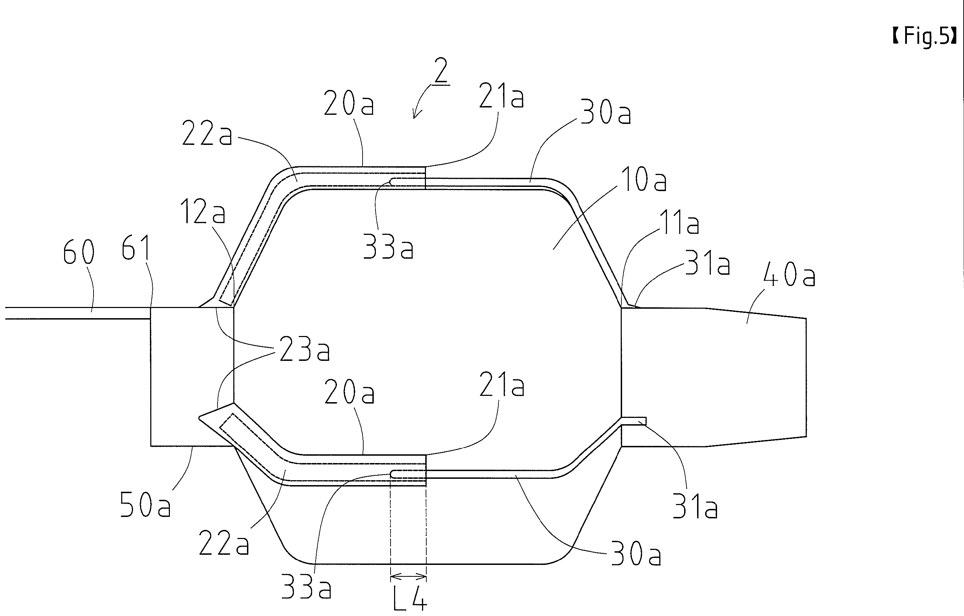

Next, a medical device 2 according to a first example is described below referring to FIG. 4 and FIG. 5. In FIG. 4 and FIG. 5, the distal end side (the front-end side) to be inserted into the body is shown to the right hand side, and the proximal end side (the rear-end side, the base-end side) to be manipulated by a handler such as a doctor is shown to the left hand side. FIG. 4 shows an expandable-and-contractible member 10a radially contracted, and FIG. 5 shows the expandable-and-contractible member 10a radially expanded.

Similarly to the medical device 1, the medical device 2 essentially comprises the expandable-and-contractible member 10a capable of radially expanding and radially contracting, an incising member 20a, a core wire 30a, a distal tip 40a, a ring 50a, and a shaft 60.

The expandable-and-contractible member 10a is made of resin material and is capable of radially expanding and radially contracting. A distal end 11a of the expandable-and-contractible member 10a is fixed to the distal tip 40a, and a proximal end 12a of the expandable-and-contractible member 10a is fixed to the ring 50a.

The incising member 20a is disposed in the longitudinal direction on the outer circumference of the expandable-and-contractible member 10a and comprises a hollow portion 22a that is open toward the distal end direction. A distal end 21a of the incising member 20a is not fixed to the expandable-and-contractible member 10a and remains unsuspended, and a proximal end 23a of the incising member 20a is fixed to the ring 50a. In other words, the incising member 20a is not fixed to the expandable-and-contractible member 10a except for its proximal end 23a being fixed to the ring 50a. In the medical device 2, similarly to the case in the medical device 1, the number of the incising member 20a on the outer circumference of the expandable-and-contractible member 10a is three.

The core wire 30a is disposed in the longitudinal direction on the outer circumference of the expandable-and-contractible member 10a, with a proximal end 33a thereof being within the hollow portion 22a of the incising member 20a. As to be described below, the proximal end 33a of the core wire 30a is not fixed within the hollow portion 22a of the incising member 20a and can slide in the longitudinal direction within the hollow portion 22a of the incising member 20a as the expandable-and-contractible member 10a expands or contracts. A distal end 31a of the core wire 30a is fixed to the distal tip 40a.

To the distal end 11a of the expandable-and-contractible member 10a and the distal end 31a of the core wire 30a, the distal tip 40a made of resin material is fixed.

To the proximal end 12a of the expandable-and-contractible member 10a and the proximal end 23a of the incising member 20a, the ring 50a made of metal material is fixed.

To the ring 50a, a distal end 61 of the shaft 60 is fixed. The shaft 60 is a thin, cylinder-like metal wire (stainless steel), extending from the ring 50a in the longitudinal direction toward the proximal end side.

The handler inserts the medical device 2 into a blood vessel or a digestive organ and manipulates the shaft 60 in the longitudinal direction to deliver the medical device 2 to the site of a stenosis or stricture. During this procedure, the expandable-and-contractible member 10a is radially contracted (see FIG. 4). Subsequently, the handler inserts a balloon catheter into the interior of the expandable-and-contractible member 10a of the medical device 2 and then makes the balloon of the balloon catheter radially expand so as to radially expand the expandable-and-contractible member 10a (see FIG. 5). By pressing the incising member 20a against the stenosis or stricture, the handler can incise the stenosis or stricture in the longitudinal direction.

In FIG. 5, the balloon catheter inserted in the interior of the expandable-and-contractible member 10a is omitted for ease of understanding. The balloon catheter used in the medical device 2 can be a known balloon catheter.

In the medical device 2, the core wire 30a, which is inserted in the hollow portion 22a of the incising member 20a from the distal end direction, can slide in the longitudinal direction within the hollow portion 22a as the expandable-and-contractible member 10a expands or contracts. More specifically, upon radial expansion of the expandable-and-contractible member 10a (in other words, upon a shift from the state in FIG. 4 to the state in FIG. 5), part of the core wire 30a housed within the hollow portion 22a of the incising member 20a becomes shorter in the longitudinal direction from L3 to L4, while, upon radial contraction of the expandable-and-contractible member 10a (in other words, upon a shift from the state in FIG. 5 to the state in FIG. 4), the part of the core wire 30a housed within the hollow portion 22a of the incising member 20a becomes longer in the longitudinal direction from L4 to L3.

As described above, in the medical device 2, the incising member 20a is not fixed to the expandable-and-contractible member 10a, and, owing to the presence of the core wire 30a inserted in the hollow portion 22a, the incising member 20a does not comes off the expandable-and-contractible member 10a as the expandable-and-contractible member 10a expands or contracts and can slide to an optimum position, consequently reducing the possibility of the incising member 20a interfering with the radial expansion or the radial contraction of the expandable-and-contractible member 10a. Even when the stenosis or stricture is partially caught between the expandable-and-contractible member 10a and the incising member 20a and the handler manipulates the medical device 2 in the longitudinal direction in that state, the core wire 30a can be pulled out of the hollow portion 22a of the incising member 20a (in other words, the core wire 30a and the incising member 20a can become separated from each other), so that the load (external force) applied on the incising member 20a by the stenosis or stricture thus caught can be reduced, consequently reducing the possibility of the incising member 20a coming off.

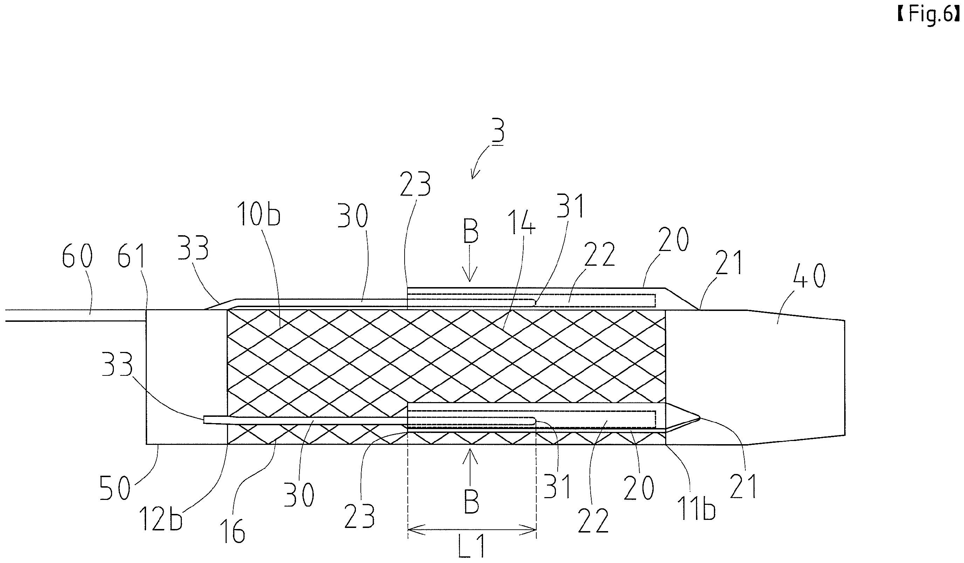

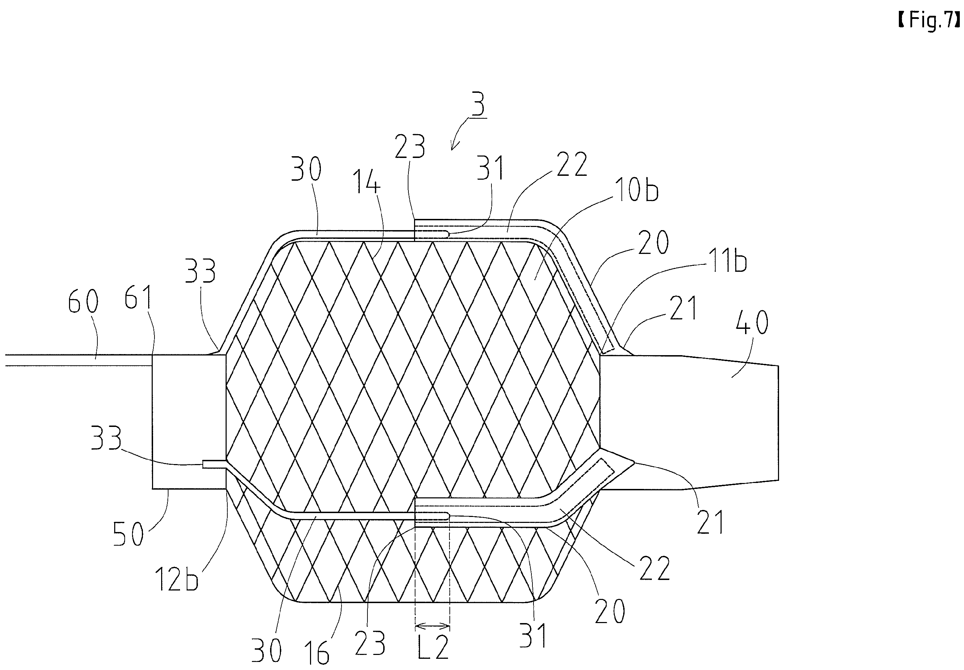

Next, a medical device 3 according to a second embodiment is described below referring to FIG. 6 to FIG. 8. In FIG. 6 and FIG. 7, the distal end side (the front-end side) to be inserted into the body is shown to the right hand side, and the proximal end side (the rear-end side, the base-end side) to be manipulated by a handler such as a doctor is shown to the left hand side. FIG. 6 shows an expandable-and-contractible member 10b radially contracted, and FIG. 7 shows the expandable-and-contractible member 10b radially expanded.

FIG. 6 is a variation of FIG. 1, and FIG. 7 is a variation of FIG. 2. Description of the medical device 3 below only includes its differences from the medical device 1. The medical device 3 comprises the expandable-and-contractible member 10b, instead of the expandable-and-contractible member 10, and the expandable-and-contractible member 10b is a mesh member having a mesh configuration (a net configuration) woven from a first wire 14 and a second wire 16 (see FIG. 6 and FIG. 7). A distal end 11b of the expandable-and-contractible member 10b is fixed to the distal tip 40, and a proximal end 12b of the expandable-and-contractible member 10b is fixed to the ring 50.

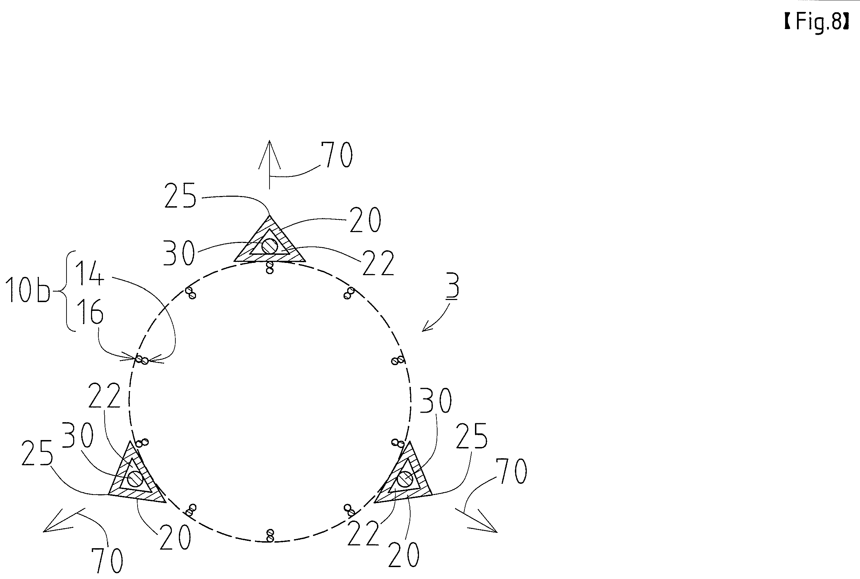

FIG. 8 is a sectional view taken from line B-B of FIG. 6. On the outer circumference of the expandable-and-contractible member 10b of the medical device 3, similarly to the case of the medical device 1, three incising members 20 are disposed in an arrangement where they are away from each other by 120 degrees in the circumferential direction. The incising member 20 is a triangular tube with its interior being the hollow portion 22, and comprises the protruding portion 25 protruding outwardly. Upon radial expansion of the expandable-and-contractible member 10b, the incising member 20 moves to the direction shown by an arrow 70, so that the protruding portion 25 of the incising member 20 can incise a stenosis or stricture.

In the medical device 3, the core wire 30, which is inserted in the hollow portion 22 of the incising member 20 from the proximal end direction, can slide in the longitudinal direction within the hollow portion 22 as the expandable-and-contractible member 10b expands or contracts. More specifically, upon radial expansion of the expandable-and-contractible member 10b (in other words, upon a shift from the state in FIG. 6 to the state in FIG. 7), part of the core wire 30 housed within the hollow portion 22 of the incising member 20 becomes shorter in the longitudinal direction from L1 to L2, while, upon radial contraction of the expandable-and-contractible member 10b (in other words, upon a shift from the state in FIG. 7 to the state in FIG. 6), the part of the core wire 30 housed within the hollow portion 22 of the incising member 20 becomes longer in the longitudinal direction from L2 to L1.

As described above, in the medical device 3, the incising member 20 is not fixed to the expandable-and-contractible member 10b, and, owing to the presence of the core wire 30 inserted in the hollow portion 22, the incising member 20 does not comes off the expandable-and-contractible member 10b as the expandable-and-contractible member 10b expands or contracts and can slide to an optimum position, consequently reducing the possibility of the incising member 20 interfering with the radial expansion or the radial contraction of the expandable-and-contractible member 10b. Even when the stenosis or stricture is partially caught between the expandable-and-contractible member 10b and the incising member 20 and the handler manipulates the medical device 1 in the longitudinal direction in that state, the core wire 30 can be pulled out of the hollow portion 22 of the incising member 20 (in other words, the core wire 30 and the incising member 20 can become separated from each other), so that the load (external force) applied on the incising member 20 by the stenosis or stricture thus caught can be reduced, consequently reducing the possibility of the incising member 20 coming off.

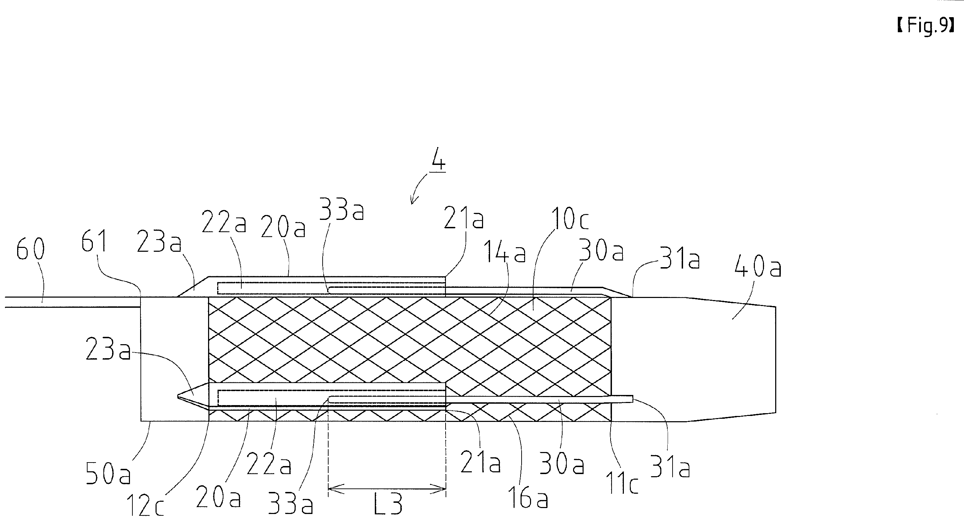

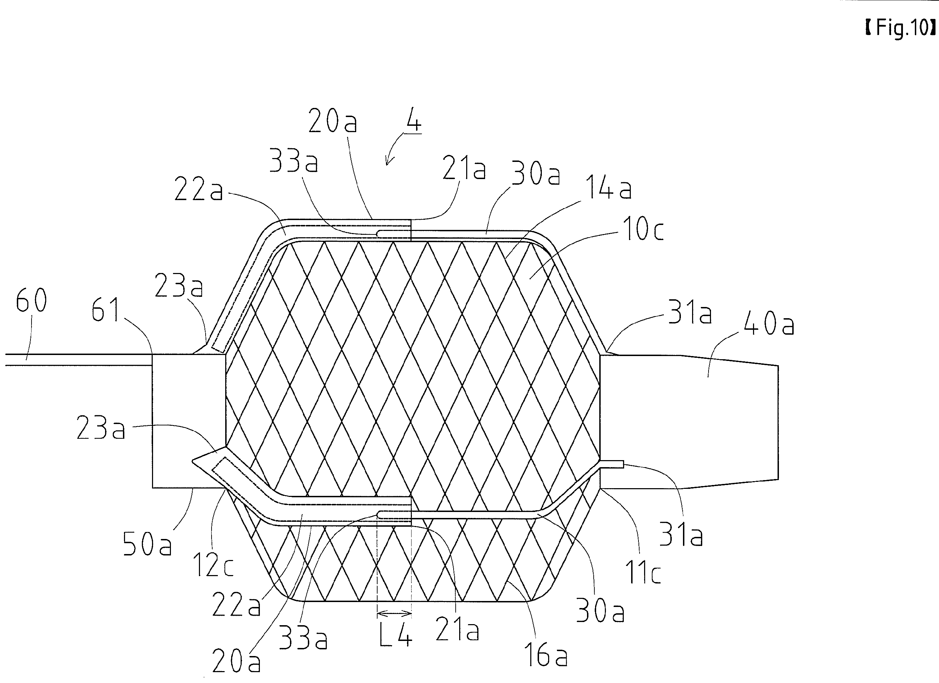

Next, a medical device 4 according to second example is described below referring to FIG. 9 and FIG. 10. In FIG. 9 and FIG. 10, the distal end side (the front-end side) to be inserted into the body is shown to the right hand side, and the proximal end side (the rear-end side, the base-end side) to be manipulated by a handler such as a doctor is shown to the left hand side. FIG. 9 shows an expandable-and-contractible member 10c radially contracted, and FIG. 10 shows the expandable-and-contractible member 10c radially expanded.

FIG. 9 is a variation of FIG. 4, and FIG. 10 is a variation of FIG. 5. Description of the medical device 4 below only includes its differences from the medical device 2. The medical device 4 comprises the expandable-and-contractible member 10c, instead of the expandable-and-contractible member 10a, and the expandable-and-contractible member 10c is a mesh member having a mesh configuration (a net configuration) woven from a first wire 14a and a second wire 16a (see FIG. 9 and FIG. 10). A distal end 11c of the expandable-and-contractible member 10c is fixed to the distal tip 40a, and a proximal end 12c of the expandable-and-contractible member 10c is fixed to the ring 50a.

In the medical device 4, the core wire 30a, which is inserted in the hollow portion 22a of the incising member 20a from the distal end direction, can slide in the longitudinal direction within the hollow portion 22a as the expandable-and-contractible member 10c expands or contracts. More specifically, upon radial expansion of the expandable-and-contractible member 10c (in other words, upon a shift from the state in FIG. 9 to the state in FIG. 10), part of the core wire 30a housed within the hollow portion 22a of the incising member 20a becomes shorter in the longitudinal direction from L3 to L4, while, upon radial contraction of the expandable-and-contractible member 10c (in other words, upon a shift from the state in FIG. 10 to the state in FIG. 9), the part of the core wire 30a housed within the hollow portion 22a of the incising member 20a becomes longer in the longitudinal direction from L4 to L3.

As described above, in the medical device 4, the incising member 20a is not fixed to the expandable-and-contractible member 10c, and, owing to the presence of the core wire 30a inserted in the hollow portion 22a, the incising member 20a does not comes off the expandable-and-contractible member 10c as the expandable-and-contractible member 10c expands or contracts and can slide to an optimum position, consequently reducing the possibility of the incising member 20a interfering with the radial expansion or the radial contraction of the expandable-and-contractible member 10c. Even when the stenosis or stricture is partially caught between the expandable-and-contractible member 10c and the incising member 20a and the handler manipulates the medical device 4 in the longitudinal direction in that state, the core wire 30a can be pulled out of the hollow portion 22a of the incising member 20a (in other words, the core wire 30a and the incising member 20a can become separated from each other), so that the load (external force) applied on the incising member 20a by the stenosis or stricture thus caught can be reduced, consequently reducing the possibility of the incising member 20a coming off.

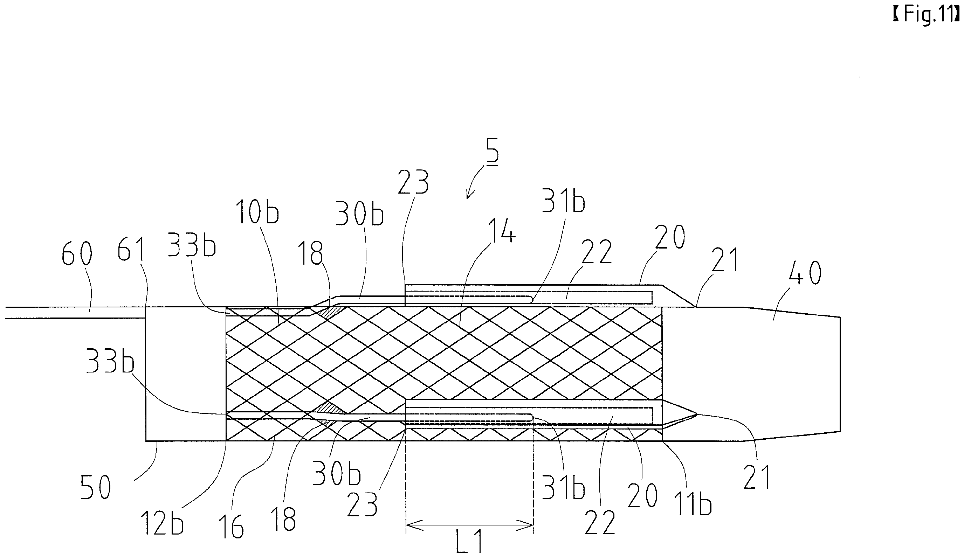

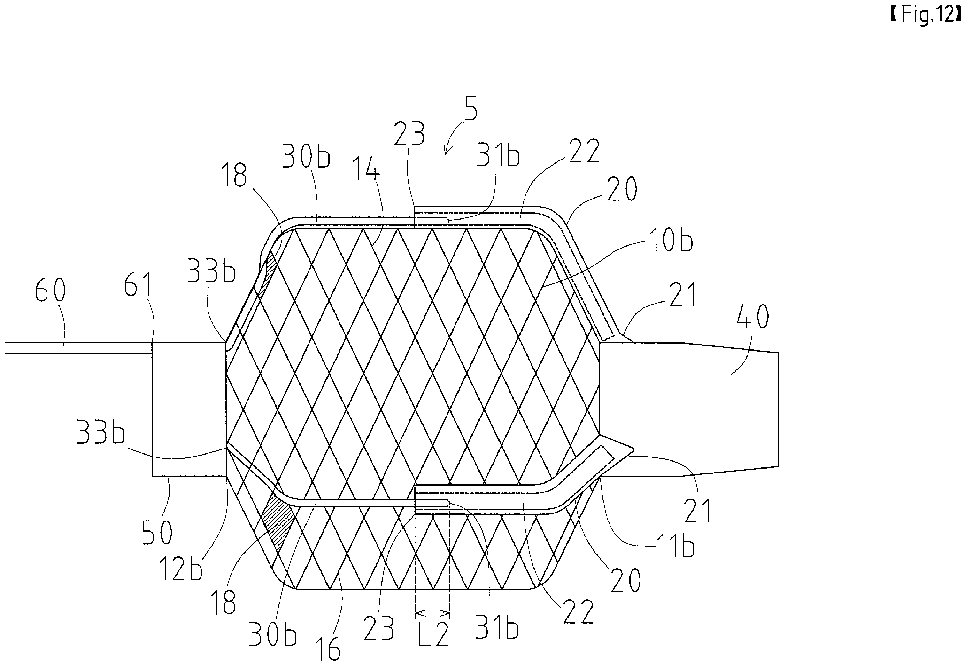

Next, a medical device 5 according to a third embodiment is described below referring to FIG. 11 and FIG. 12. In FIG. 11 and FIG. 12, the distal end side (the front-end side) to be inserted into the body is shown to the right hand side, and the proximal end side (the rear-end side, the base-end side) to be manipulated by a handler such as a doctor is shown to the left hand side. FIG. 11 shows the expandable-and-contractible member 10b radially contracted, and FIG. 12 shows the expandable-and-contractible member 10b radially expanded.

FIG. 11 is a variation of FIG. 6, and FIG. 12 is a variation of FIG. 7. Description of the medical device 5 below only includes its differences from the medical device 3. In the medical device 5, a core wire 30b extends in the distal end direction coming from inside the expandable-and-contractible member 10b to the outside at one of spaces 18 defined by the first wire 14 and the second wire 16, as seen from a proximal end 33b of the core wire 30b toward a distal end 31b of the core wire 30b. In other words, as seen from the distal end 31b toward the proximal end 33b, the core wire 30b extends in the proximal end direction going underneath part of the spaces 18 defined by the first wire 14 and the second wire 16, and then, at the proximal end 33b, it is fixed to the ring 50.

Similarly to the case of the medical device 3, the core wire 30b, which is inserted in the hollow portion 22 of the incising member 20 from the proximal end direction, can slide in the longitudinal direction within the hollow portion 22 as the expandable-and-contractible member 10b expands or contracts. More specifically, upon radial expansion of the expandable-and-contractible member 10b (in other words, upon a shift from the state in FIG. 11 to the state in FIG. 12), part of the core wire 30b housed within the hollow portion 22 of the incising member 20 becomes shorter in the longitudinal direction from L1 to L2, while, upon radial contraction of the expandable-and-contractible member 10b (in other words, upon a shift from the state in FIG. 12 to the state in FIG. 11), the part of the core wire 30b housed within the hollow portion 22 of the incising member 20 becomes longer in the longitudinal direction from L2 to L1.

In the medical device 5, in which only a short part of the core wire 30b sticks out of the expandable-and-contractible member 10b from one of the spaces 18, even if the core wire 30b is pulled out of the hollow portion 22 of the incising member 20 (in other words, the core wire 30b and the incising member 20 become separated from each other), the likelihood of the core wire 30b moving outwardly and coming off the expandable-and-contractible member 10b is reduced, and, as a result, the possibility of the wall of a normal blood vessel or the wall of a normal digestive organ becoming impaired by the core wire 30b pulled out of (or separated from) the incising member 20 can be reduced.

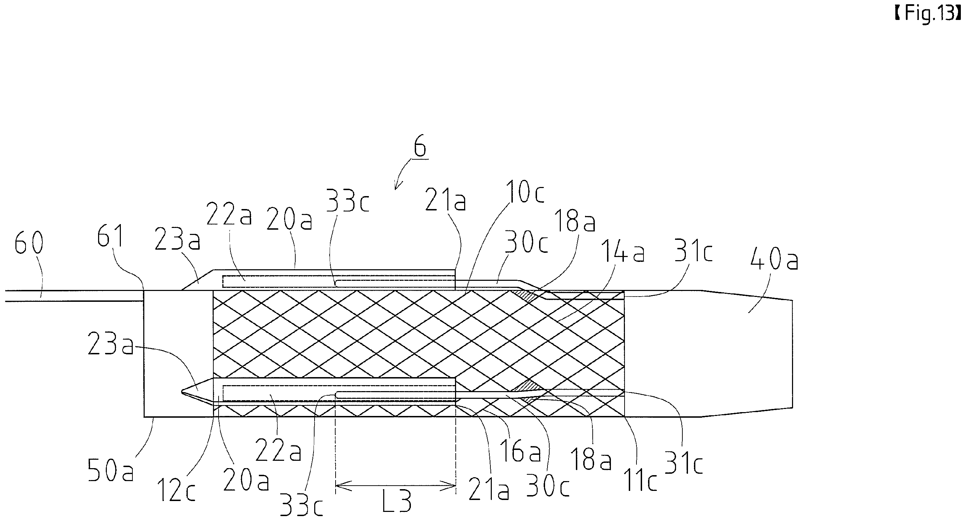

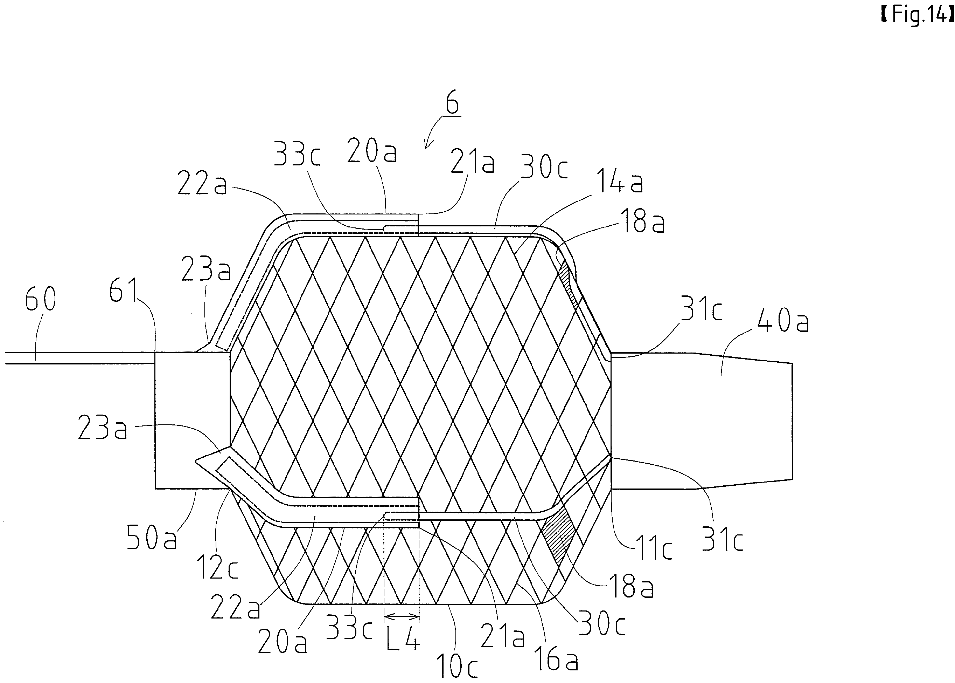

Next, a medical device 6 according to a third example described below referring to FIG. 13 and FIG. 14. In FIG. 13 and FIG. 14, the distal end side (the front-end side) to be inserted into the body is shown to the right hand side, and the proximal end side (the rear-end side, the base-end side) to be manipulated by a handler such as a doctor is shown to the left hand side. FIG. 13 shows the expandable-and-contractible member 10c radially contracted, and FIG. 14 shows the expandable-and-contractible member 10c radially expanded.

FIG. 13 is a variation of FIG. 9, and FIG. 14 is a variation of FIG. 10. Description of the medical device 6 below only includes its differences from the medical device 4. In the medical device 6, a core wire 30c extends in the proximal end direction coming from inside the expandable-and-contractible member 10c to the outside at one of spaces 18a defined by the first wire 14a and the second wire 16a, as seen from a distal end 31c of the core wire 30c toward a proximal end 33c of the core wire 30c. In other words, as seen from the proximal end 33c toward the distal end 31c, the core wire 30c extends in the distal end direction going underneath part of the spaces 18a defined by the first wire 14a and the second wire 16a, and then, at the distal end 31c, it is fixed to the distal tip 40a.

Similarly to the case of the medical device 4, the core wire 30c, which is inserted in the hollow portion 22a of the incising member 20a from the distal end direction, can slide in the longitudinal direction within the hollow portion 22a as the expandable-and-contractible member 10c expands or contracts. More specifically, upon radial expansion of the expandable-and-contractible member 10c (in other words, upon a shift from the state in FIG. 13 to the state in FIG. 14), part of the core wire 30c housed within the hollow portion 22a of the incising member 20a becomes shorter in the longitudinal direction from L3 to L4, while, upon radial contraction of the expandable-and-contractible member 10c (in other words, upon a shift from the state in FIG. 14 to the state in FIG. 13), the part of the core wire 30c housed within the hollow portion 22a of the incising member 20a becomes longer in the longitudinal direction from L4 to L3.

In the medical device 6, in which only a short part of the core wire 30c sticks out of the expandable-and-contractible member 10c from one of the spaces 18a, even if the core wire 30c is pulled out of the hollow portion 22a of the incising member 20a (in other words, the core wire 30c and the incising member 20a become separated from each other), the likelihood of the core wire 30c moving outwardly and coming off the expandable-and-contractible member 10c is reduced, and, as a result, the possibility of the wall of a normal blood vessel or the wall of a normal digestive organ becoming impaired by the core wire 30c pulled out of (or separated from) the incising member 20a can be reduced.

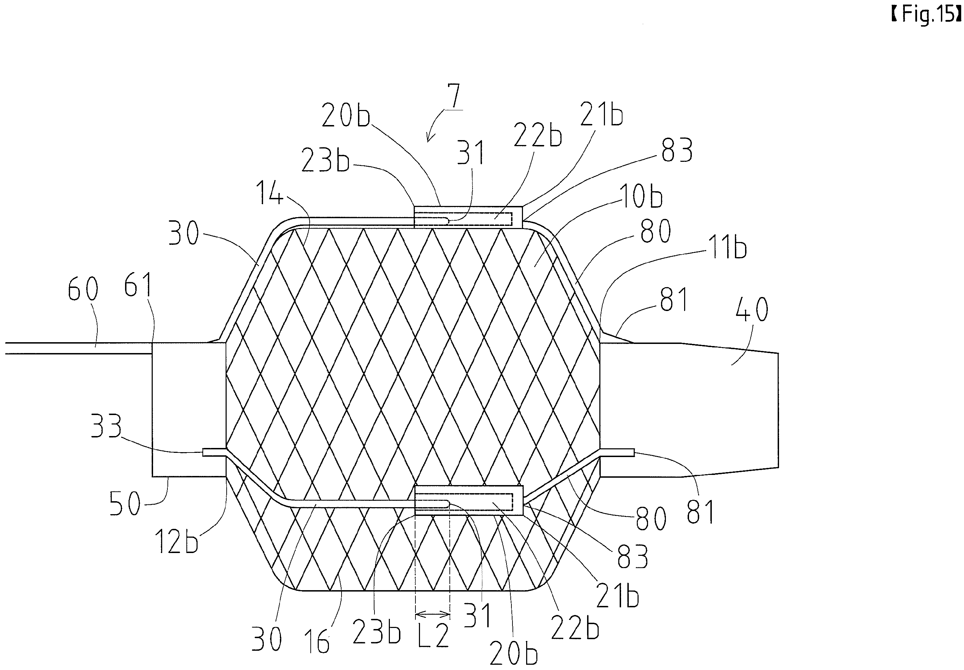

In the description of the medical devices 1, 3, and 5, the distal end 21 of the incising member 20 is directly fixed to the distal tip 40, but this configuration is not limitative. For example, as a variation of the medical device 3 in FIG. 7, a medical device 7 according to a fourth embodiment has a configuration shown in FIG. 15. In FIG. 15, a connecting member 80 is provided between a distal end 21b of an incising member 20b and the distal tip 40. More specifically, the distal end 21b of the incising member 20b is fixed to a proximal end 83 of the connecting member 80, and a distal end 81 of the connecting member 80 is fixed to the distal tip 40. The proximal end 23b of the incising member 20b is not fixed to the expandable-and-contractible member 10b and remains unsuspended.

In the medical device 7, similarly to the case in the medical device 3, the core wire 30 inserted in a hollow portion 22b of the incising member 20b from the proximal end direction can slide in the longitudinal direction within the hollow portion 22b as the expandable-and-contractible member 10b expands or contracts.

In the medical devices 2, 4, and 6, the proximal end 23a of the incising member 20a is directly fixed to the ring 50a, but this configuration is not limitative. Alternatively, a connecting member 80 (not shown) may be provided between the incising member 20a and the ring 50a.

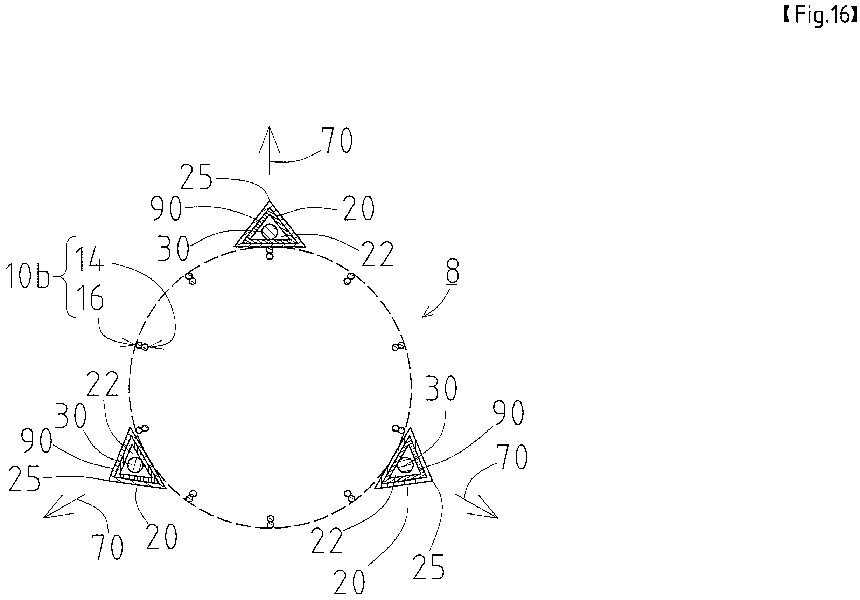

In the descriptions of the medical devices 1 to 7, the incising member 20, 20a, or 20b is made of monolayer material, but it is not limitative. For example, as a variation of the medical device 3 in FIG. 8, FIG. 16 shows a medical device 8 according to an fifth embodiment. In FIG. 16, the inner surface of the incising member 20 can be covered with a resin layer 90 with low sliding resistance (PTFE or polyimide, for example) so that the core wire 30 inserted in the hollow portion 22 of the incising member 20 can easily slide in the longitudinal direction as the expandable-and-contractible member 10b expands or contracts.

In the medical device 1 or 2 according to the first embodiment or the first example, the expandable-and-contractible member 10 or 10a is made of polyamide, but it is not limitative. Alternatively, the expandable-and-contractible member 10 or 10a may be made of a resin material such as polyester, polyurethane, polyolefin, polytetrafluoroethylene, or a silicon resin.

In the expandable-and-contractible member 10b or 10c of the medical devices 3 to 8 according to the second embodiment to the fifth embodiment or the first example to the third example, the first wire 14 and the second wire 16 are made of a Co-Cr alloy with high tensile strength, but it is not limitative. For example, the first wire 14 and the second wire 16 may be made of a metal material such as stainless steel, W, Pt, a Pt-Ni alloy, an Ni-Ti alloy, or a Cu-Al-Ni alloy. The material of the first wire 14 may be different from the material of the second wire 16. Preferably, at least one of the first wire 14 and the second wire 16 is made of a radiopaque material (tungsten, for example) because, in that case, imaging allows the handler to see exactly where the expandable-and-contractible member 10b or 10c is and consequently to easily place the expandable-and-contractible member 10b or 10c in the site of a stenosis or stricture.

In the medical devices 1 to 8 according to the first embodiment to the fifth embodiment or the first example to the third example, the incising member 20, 20a, or 20b and the core wire 30, 30a, 30b, or 30c are made of stainless steel, but it is not limitative. Alternatively, the incising member 20, 20a, or 20b and the core wire 30, 30a, 30b, or 30c may be made of a metal material such as W, Pt, a Pt-Ni alloy, an Ni-Ti alloy, or a Cu-Al-Ni alloy or a hard resin material such as polyimide or PEEK (polyether ether ketone).

The number of the incising member 20, 20a, or 20b and the number of the core wire 30, 30a, 30b, or 30c are not limited to three. At least one incising member 20, 20a, or 20b and at least one core wire 30, 30a, 30b, or 30c are simply required to be disposed on the outer circumference of the expandable-and-contractible member 10, 10a, 10b, or 10c.

### [Reference Signs List]

1, 2, 3, 4, 5, 6, 7, 8: Medical device
10, 10a, 10b, 10c: Expandable-and-contractible member
11, 11a, 11b, 11c: Distal end of expandable-and-contractible member
12, 12a, 12b, 12c: Proximal end of expandable-and-contractible member
14: First elemental thread
16: Second elemental thread
18, 18a: Space
20, 20a, 20b: Incising member
21, 21a, 21b: Distal end of incising member
22, 22a, 22b: Hollow portion
23, 23a, 23b: Proximal end of incising member
30, 30a, 30b, 30c: Core wire
31, 31a, 31b, 31c: Distal end of core wire
33, 33a, 33b, 33c: Proximal end of core wire
40, 40a: Distal tip
50, 50a: Ring
60: Shaft
70: Arrow
80: Connecting member
90: Resin layer

## Claims

1. A medical device (1, 3, 5, 7, 8) having a distal end and a proximal end and comprising:
a tubular expandable-and-contractible member (10, 10b) capable of radially expanding and radially contracting;
an incising member (20, 20b) disposed on an outer circumference of the expandable-and-contractible member (10, 10b) and comprising a hollow portion (22, 22b) open toward the proximal end direction;
a core wire (30, 30b) inserted in the hollow portion of the incising member (20, 20b) from the proximal end direction;
a distal tip (40) fixed to both a distal end (11, 11b) of the expandable-and-contractible member (10, 10b) and a distal end (21, 21b) of the incising member (20, 20b); and
a ring (50) fixed to both a proximal end (12, 12b) of the expandable-and-contractible member (10, 10b) and a proximal end (33, 33b) of the core wire (30, 30b),
wherein the core wire (30, 30b) is capable of sliding in a longitudinal direction within the hollow portion (22, 22b) as the expandable-and-contractible member (10, 10b) expands or contracts, **characterized in that**
the incising member (20, 20b) is not fixed to the expandable-and-contractible member (10, 10b).

2. The medical device (5) according to claim 1,
wherein the expandable-and-contractible member (10b) is a mesh member woven from a first wire (14, 14a) and a second wire (16, 16a), and
wherein the core wire (30b) extends in the longitudinal direction underneath a space (18, 18a) that is defined by the first wire (14, 14a) and the second wire (16, 16a).

3. The medical device (7) according to claim 1 or 2,
wherein a proximal end of a connecting member (80) is fixed to the distal end (21b) of the incising member (20b) and a distal end of the connecting member (80) is fixed to the distal tip (40).

4. The medical device (8) according to any one of claims 1 to 3,
wherein the inner surface of the incising member (20) is covered with a resin layer (90) with low sliding resistance.

## Patentansprüche

1. Medizinische Vorrichtung (1, 3, 5, 7, 8) mit einem distalen Ende und einem proximalen Ende und:
einem tubulären dehnfähigen/kontraktilen Element (10, 10b), das in der Lage ist, sich radial auszudehnen und radial zusammenzuziehen;
einem am Außenumfang des dehnfähigen/kontraktilen Elements (10, 10b) angeordneten Schneidelement (20, 20b), das einen zu dem proximalen Ende hin offenen hohlen Abschnitt (22, 22b) aufweist;
einem Kerndraht (30, 30b), der von dem proximalen Ende her in dem hohlen Abschnitt des Schneidelements (20, 20b) steckt;
einer distalen Spitze (40), die sowohl an einem distalen Ende (11, 11b) des dehnfähigen/kontraktilen Elements (10, 10b) als auch an einem distalen Ende (21, 21b) des Schneidelements (20, 20b) befestigt ist; und
einem Ring (50), der sowohl an einem proximalen Ende (12, 12b) des dehnfähigen/kontraktilen Elements (10, 10b) als auch an einem proximalen Ende (33, 33b) des Kerndrahts (30, 30b) befestigt ist,
wobei der Kerndraht (30, 30b) in der Lage ist, in dem hohlen Abschnitt (22, 22b) in Längsrichtung zu gleiten, wenn sich das dehnfähige/kontraktile Element (10, 10b) ausdehnt oder zusammenzieht, **dadurch gekennzeichnet, dass**
das Schneidelement (20, 20b) an dem dehnfähigen/kontraktilen Element (10, 10b) nicht befestigt ist.

2. Medizinische Vorrichtung (5) nach Anspruch 1,
wobei das dehnfähige/kontraktile Element (10b) ein aus einem ersten Draht (14, 14a) und einem zweiten Draht (16, 16a) hergestelltes Geflecht ist, und
wobei sich der Kerndraht (30b) in der Längsrichtung unter einen durch den ersten Draht (14, 14a) und zweiten Draht (16, 16a) definierten Raum (18, 18a) erstreckt.

3. Medizinische Vorrichtung (7) nach Anspruch 1 oder 2, wobei ein proximales Ende eines Verbindungselements (80) an dem distalen Ende (21b) des Schneidelements (20b) und ein distales Ende des Verbindungselements (80) an der distalen Spitze (40) befestigt ist.

4. Medizinische Vorrichtung (8) nach einem der Ansprüche 1 bis 3,
wobei die Innenoberfläche des Schneidelements (20) von einer Harzschicht (90) mit geringem Gleitwiderstand bedeckt ist.

## Revendications

1. Dispositif médical (1, 3, 5, 7, 8) ayant une extrémité distale et une extrémité proximale et comprenant :
un élément tubulaire extensible et contractable (10, 10b) capable de s'étendre radialement et de se contracter radialement ;
un élément d'incision (20, 20b) disposé sur une circonférence extérieure de l'élément extensible et contractable (10, 10b) et comprenant une partie creuse (22, 22b) ouverte vers la direction d'extrémité proximale ;
un fil central (30, 30b) inséré dans la partie creuse de l'élément d'incision (20, 20b) à partir de la direction d'extrémité proximale ;
une pointe distale (40) fixée à la fois à une extrémité distale (11, 11b) de l'élément extensible et contractable (10, 10b) et à une extrémité distale (21, 21b) de l'élément d'incision (20, 20b) ; et
une bague (50) fixée à la fois à une extrémité proximale (12, 12b) de l'élément extensible et contractable (10, 10b) et à une extrémité proximale (33, 33b) du fil central (30, 30b),
dans lequel le fil central (30, 30b) est capable de coulisser dans une direction longitudinale à l'intérieur de la partie creuse (22, 22b) lorsque l'élément extensible et contractable (10, 10b) s'étend ou se contracte, **caractérisé en ce que**
l'élément d'incision (20, 20b) n'est pas fixé à l'élément extensible et contractable (10, 10b).

2. Dispositif médical (5) selon la revendication 1,
dans lequel l'élément extensible et contractable (10b) est un élément en treillis tissé à partir d'un premier fil (14, 14a) et d'un second fil (16, 16a), et
dans lequel le fil central (30b) s'étend dans la direction longitudinale sous un espace (18, 18a) qui est défini par le premier fil (14, 14a) et le second fil (16, 16a).

3. Dispositif médical (7) selon la revendication 1 ou 2,
dans lequel une extrémité proximale d'un élément de connexion (80) est fixée à l'extrémité distale (21b) de l'élément d'incision (20b) et une extrémité distale de l'élément de connexion (80) est fixée à la pointe distale (40).

4. Dispositif médical (8) selon l'une quelconque des revendications 1 à 3,
dans lequel la surface interne de l'élément d'incision (20) est recouverte d'une couche de résine (90) à faible résistance au glissement.
